# EUROPÄISCHE PATENTSCHRIFT

(11) **EP 1 082 049 B1**
(45) Veröffentlichungstag und Bekanntmachung des Hinweises auf die Patenterteilung: **25.09.2002**
(21) Anmeldenummer: 00925059.8
(22) Anmeldetag: 30.03.2000
(51) Int. Cl.: A61B 3/135

(54) **ANORDNUNG ZUR ERMITTLUNG UND REGELUNG DER LICHTSTÄRKE FÜR SPALTLAMPEN UND SPALTLEUCHTENPROJEKTOREN**
SYSTEM FOR DETERMINING AND CONTROLLING LIGHT INTENSITY FOR SLIT LAMPS AND SLIT-LAMP PROJECTORS
DISPOSITIF POUR DETERMINER ET REGULER L'INTENSITE LUMINEUSE DE LAMPES A FENTE ET DE PROJECTEURS A LAMPES A FENTE

(30) Priorität: 30.03.1999 DE 19914495
(43) Veröffentlichungstag der Anmeldung: 14.03.2001
(73) Patentinhaber: G. Rodenstock Instrumente GmbH, 40231 Düsseldorf (DE)
(72) Erfinder: REIS, Werner, D-80992 München (DE)
(74) Vertreter: Cohausz & Florack
(86) Internationale Anmeldenummer: DE0000954
(87) Internationale Veröffentlichungsnummer: WO00059367

(56) Entgegenhaltungen:
- US-A- 3 830 562
- US-A- 4 877 321
- US-A- 5 686 981

## Beschreibung

### Technisches Gebiet

Die Erfindung bezieht sich auf eine Anordnung zur Ermittlung und Regelung der Lichtstärke für Spaltlampen oder Spaltleuchtenprojektoren.

### Stand der Technik

Es sind Spaltlampen bzw. Spaltleuchtenprojektoren bekannt, die aus einem Mikroskop und einem schwenkbaren Beleuchtungssystem bestehen, welches ein Spaltbild erzeugt. Sie dienen der vergrößerten Beobachtung des Auges und seiner Umgebung.
Bekannt sind u.a. solche Lösungen, wobei die Optik des Spaltbildprojektors gewissermaßen eine Weiterentwicklung der bis dahin bekannten Spaltbildprojektoren darstellt. Dabei wurde erstmals der Versuch unternommen, die Kondensor-Optik mit der Objektiv-Optik zu einer sehr leistungsfähigen Einheit zu verschmelzen. Der Kondensor erfüllt dabei die Aufgabe, die Glühlampenwendel in das Objektiv bzw. das Austrittsprisma abzubilden. Das Objektiv der Leuchte übernimmt dabei die Aufgabe, die Spalt- und Irisblende in die Drehachse des Gerätes, also auf das Auge zu projizieren. Weiter besitzen diese Geräte folgende Baugruppen, das die Spalt- und Irisblende abbildende Objektiv mit Umlenkprisma, variable Aperturblende, bestimmte variable Filteranordnungen, Ausgleichsglasplatte sowie das Gehäuse und die Verstellmechanik.
Das Ziel der bisherigen Entwicklungen von Spaltlampen ist von der Erreichung folgender Gebrauchseigenschaften geprägt:
- excellente Abbildungsqualität,
- akkomodationsfreie Beobachtung
- mehrere Vergrößerungsstufen
- extrem hohe Beleuchtungsstärke
- optisch umschaltbare Spaltlängen
- serienmäßige Brillenträgerokulare
- excellente Spaltbilder dank sorgfältig abgestimmter Apertur- und Lichtverhältnisse
- hohe Beleuchtungsstärke und konstante Leuchtdichte
- Übersichtsbetrachtungen bei diffuser Beleuchtung mit vorschwenkbarem Mattglas
- gestochen scharfe Spaltbilder von der Hornhautvorderfläche bis zur Linsenrückfläche durch austauschbaren Prismenkopf mit variabler Apertur

Aus der DE 195 39 371 A1 ist ein abbildendes optisches Gerät bekannt, dessen Aufgabe es ist, die Bereitstellung eines neuartigen, vergrößernden oder verkleinernden optischen Gerätes mit verbesserten Eigenschaften, welches auch bei der Beobachtung durch trübe Medien ein Bild des Objektes erzeugt, das möglichst kontraststark und blendfrei ist und dadurch einzelne Details sichtbar werden. Die Lichtstärke des Gerätes und die erreichte Auflösung sind dabei sehr hoch.
Eine Einrichtung zur Anzeige und Verhinderung von Überbelastung des zu untersuchenden Auges mit zu hoher Lichtstrahlung ist nicht vorgesehen.

Aus der DE 42 27 942 C2 ist eine Spaltlampen-Beleuchtungseinrichtung zur Abbildung einer Spaltblende in eine Zielebene bekannt, die aus folgenden Bestandteilen besteht:
- einer Lichtquelle, vorzugsweise hoher Intensität, der ein faseroptischer Lichtleiter nachgeordnet ist,
- einer der Lichtleiter-Austrittsfläche in geringem Abstand ohne zwischengeschalteter optischer Elemente nachgeordneten Spaltblende, sowie
- einer Spaltblende nachgeordneten Abbildungseinrichtung, mit mindestens zwei optischen Systemen mit jeweils sammelnder optischen Wirkung,
   von denen das erste so dimensioniert ist, daß eine Abbildung der Lichtleiteraustrittsfläche in eine Ebene erfolgt, die zwischen der Zielebene und der Hauptebene des bildnächsten optischen Systemes liegt und gleichzeitig mit dem ersten optischen System eine Kollimation des Spaltblenden-Abbildungs-Strahlenganges erfolgt,
   während das zweite optische System eine Fokussierung des kollimierten Spalt-Abbildungs-Strahlenganges in die Zielebene bewirkt, wodurch eine vollständige Ausnutzung der numerischen Apertur des faseroptischen Lichtleiters durch die Abbildungseinrichtung resultiert.

Eine Einrichtung zur Anzeige und Vermeidung von lichttechnischer Überbelastung ist auch hier nicht vorgesehen.

Aus US-A-4877321 ist eine Spaltlampe mit einem Lichtdetektor, der das vom ange reflektierten Licht mipt und ein signal abgibt das zur Lichtstärke Regelung verwendet wird, bekannt.

In der europäischen Norm DIN EN ISO 10939 sind u.a. die Anforderungen an Spaltleuchten und Spaltleuchtenprojektoren geregelt. Im Abschnitt 4. dieser Norm sind die Anforderungen und im Abschnitt 4.4 besonders die Anforderungen hinsichtlich der Gefährdung durch optische Strahlung bei Spaltleuchten definiert. Dort sind Grenzwerte festgelegt und speziell im Abschnitt 4.4.4. folgende Forderungen vorgegeben:
"Der Hersteller muß dem Anwender eine Grafik zur Verfügung stellen, aus der die von der Spaltleuchte abgegebene relative spektrale Strahlungsintensität im Bereich zwischen 305 nm und 1.100 nm hervorgeht, für den Fall, daß das Instrument mit maximaler Lichtintensität und bei maximaler Apertur betrieben wird. Die austretende spektrale Strahlungsintensität muß für das Strahlungsbündel angegeben werden, nachdem es aus dem Instrument ausgetreten ist.
Der Hersteller muß dem Anwender die Werte der spektralbewerteten photochemischen Strahldichte und der Strahlungsquelle sowohl für das phakische Auge L_{B} als auch für das aphakische Auge L_{A} angeben. Diese muß im aus dem Instrument austretenden Bündel bestimmt werden für den Fall, daß das Instrument bei maximaler Lichtintensität und maximaler Apertur betrieben wird."
Weiter ist in dieser DIN gefordert, daß der Hersteller dem Nutzer Begleitdokumente z.B. Gebrauchsanweisungen zu übergeben hat, die u.a. auch die Anforderungen gemäß 4.4.4. der DIN EN ISO 10939 erfüllen muß.
Dies geschieht in der Praxis derzeit durch die Beigabe entsprechender Diagramme aus denen sich z.B. die Bestrahlungsstärke im Verhältnis zur angelegten Spannung ergibt. Dabei können kurzzeitige Überbeanspruchungen des zu untersuchenden Auges nicht ausgeschlossen werden. Die einfachste Lösung wäre eine generelle Absenkung der Beleuchtungsstärke der Spaltlampen, wobei ein Hauptargument beim Einsatz der Spaltleuchten allerdings gerade die hohe Lichtstärke des Spaltprojektors ist.

So genügen die derzeit praktizierten Methoden und Mittel der Ermittlung und Beeinflussung der Lichtstärke an Spaltlampen und Spaltleuchtenprojektoren zwar den Anforderungen der DIN, eine kurzzeitige Überbelastung des untersuchten Auges durch zu hohe Lichtbelastung kann jedoch nicht ausgeschlossen werden.

Es besteht daher die Aufgabe, eine Lösung zu entwickeln, die einerseits das Arbeiten am zu untersuchenden Auge mit hoher Ausleuchtung erlaubt, eine Schädigung des Probantenauges bei Untersuchungen mit der Spaltlampe jedoch verhindert, ohne die maximale Höhe der Lichtstärke der Spaltleuchte generell zu reduzieren.

### Darstellung der Erfindung

Erfindungsgemäß wird die Aufgabe durch eine Anordnung zur Ermittlung und Regelung der Lichtstärke für Spaltlampen oder Spaltleuchtenprojektoren derart gelöst, daß in einer Spaltlampe oder einem Spaltlampenmikroskop eine schrägstehende, dünne Glasplatte mit einer Teilreflexion des Lichtes in einem definierten Winkel zum Beleuchtungsstrahlengang oberhalb der Filterbaugruppe zwischen dieser und dem nicht geteilten Objektiv Spaltprojektor derart angeordnet ist, daß ein Teil der auftreffenden Strahlen als abgelenktes Strahlenbündel auf einen seitlich im Strahlengang am Gehäuse angeordneten Detektor bzw. eine Detektorbaugruppe trifft, der die jeweils vorhandene Lichtstärke mißt, die ermittelten Werte zu einer Auswerte- und Steuereinrichtung überträgt, diese die ankommenden Werte mit einem vorgegebenen Höchstwert vergleicht, die Bestrahlungsdosis für das phake oder aphake Auge errechnet und bei Überschreitung des Wertes dieses an einer an sich bekannten Anzeige-Alarmeinrichtung anzeigt und/oder die Lichtstärke automatisch auf den vorgegebenen Höchstwert zurückregelt. Der Ort der Glasplatte ist deshalb so wichtig, da der Strahlengang die Größe der Wendelabbildung der Halogenlampe nicht verändert, sondern lediglich verstärkt oder abschwächt. Wird also z.B. die Spaltbeleuchtung in der Spaltbreite oder Höhe verändert, so wirkt sich das am Detektor nur durch eine Helligkeitsänderung aus. Das Spaltlampenwendel bleibt in Größe und Lage unverändert erhalten. Der Strahlengang hat an dieser Stelle eine rechteckige Lichtverteilung, so daß eine Unterbringung des seitlich angeordneten Detektors kein Problem darstellt. Die Verbindung der Detektorbaugruppe mit der Helligkeitsregelung des Spaltprojektors ist deshalb besonders günstig, da die Filterabsorption durch den gewählten Standort einbezogen wird. Der für die Anwendung uninteressante IR- und UV-Anteil könnte mit geeigneten Glassorten oder Filtern ausgeblendet werden.

In einer weiteren Ausführungsform ist die Anordnung zur Ermittlung und Regelung der Lichtstärke für Spaltlampen oder Spaltleuchtenprojektoren für Geräte mit Doppelachromat (geteilten Objektiven) und dadurch parallelem Strahlengang dadurch gekennzeichnet, daß in einer Spaltlampe oder einem Spaltlampenprojektor eine schrägstehende, dünne Glasplatte mit einer Teilreflexion des Lichtes in einem definierten Winkel von zum Strahlengang zwischen den Linsenbaugruppen des Doppelachromaten im parallelen Strahlengang angeordnet ist, dadurch ein Teil der auftreffenden Strahlen als abgelenktes Strahlenbündel auf eine seitlich im Strahlengang am Gehäuse angeordnete Detektorbaugruppe trifft, welche die jeweils vorhandene Lichtstärke mißt, die ermittelten Werte zu einer Auswerte- und Steuereinrichtung überträgt, diese die ankommenden Werte mit einem vorgegebenen Höchstwert vergleicht die Bestrahlungsdosis für das phake oder aphake Auge errechnet und bei Überschreitung des Wertes dieses an einer an sich bekannten Anzeige-, Arlarmeinrichtung anzeigt und/oder die Lichtstärke automatisch auf den vorgegebenen Höchstwert zurückregelt.

Da die hohe Beleuchtung hauptsächlich bei kleinen Spaltbreiten benötigt wird, ist der Vorteil dieser Lösung, die Gesamthelligkeit am Detektor bleibt niedrig. Beim Öffnen des Spaltes oder bei Benutzung der Filter wird generell die Beleuchtung (starke Blendung des Probanden) zurückgedreht, die Gesamthelligkeit wird damit ebenfalls reduziert. Es wird bei normaler Spaltlampenanwendung die Lichtbelastung nicht überschritten.

Die Erfindung wird nachstehend ohne Beschränkung des allgemeinen Erfindungsgedankens anhand von Ausführungsbeispielen unter Bezugnahme auf die Zeichnungen exemplarisch beschrieben, auf die im übrigen hinsichtlich der Offenbarung aller im Text nicht näher erläuterten erfindungsgemäßen Einzelheiten ausdrücklich verwiesen wird.

### Darstellung von Ausführungsbeispielen

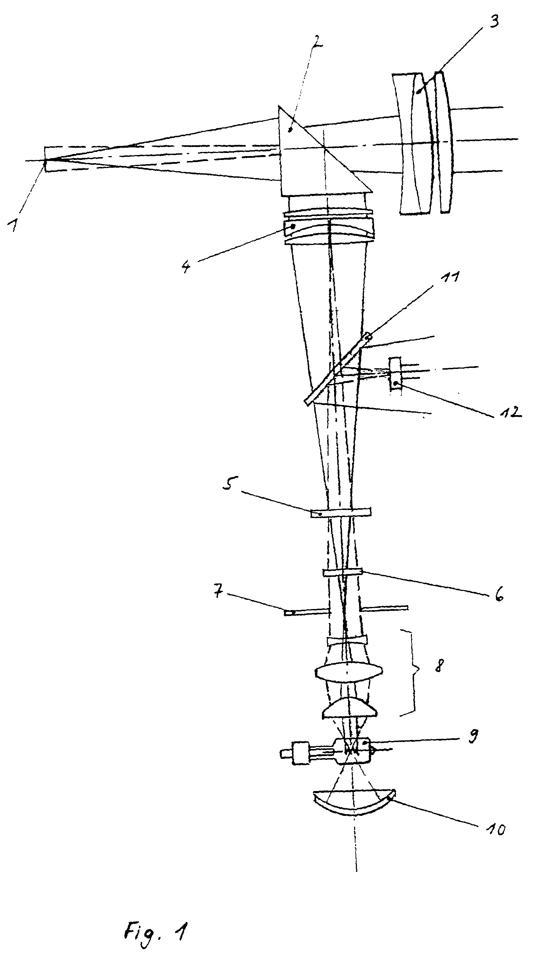

Fig. 1 zeigt eine Spaltlampe in der Ausführung mit einem Objektiv Spaltprojektor 4. Dabei sind das Spaltbild 1, ein Umlenkprisma 2, das Objektiv Stereomikroskop 3, das Objektiv Spaltprojektor 4, die Filterbaugruppe, bestehend aus einem Farbneutralglasfilter 5 und UV-Absorbtionsfilter 6, die Spaltblende 7, ein Kondensor mit IR-Absorbtion 8, eine Halogenlampe 9 und ein Hohlspiegel 10 in für diese Bauart bekannter Weise angeordnet. Erfindungsgemäß ist in einer Spaltlampe oder einem Spaltlampenprojektor dieser Bauart eine schrägstehende, dünne Glasplatte 11 mit einer Gesamtreflexion von ca. 2 Prozent in einem Winkel von 45 Grad zum Strahlengang oberhalb der Filtergruppe, bestehend aus Farbneutralfilter 5 und UV-Absorbtionsfilter 6 zwischen dieser und dem Objektiv Spaltprojektor 4 angeordnet. Dadurch wird ein Teil der auftreffenden Strahlen als abgelenktes Strahlenbündel auf einen seitlich im rechten Winkel zur Mittelachse des Strahlenganges im Gehäuse angeordneten Detektor 12 bzw. eine Detektorbaugruppe gelenkt. Der Detektor 12 oder die Detektorbaugruppe mißt die jeweils vorhandene Lichtstärke, überträgt die ermittelten Werte zu einer nicht im Bild gezeigten Auswerte- und Steuereinrichtung. Diese vergleicht die ankommenden Werte mit einem vorgegebenen Höchstwert und ermittelt die Bestrahlungsdosis für das phake oder aphake Auge. Bei Überschreitung des Wertes wird dies an einer an sich bekannten Anzeige-, Arlarmeinrichtung anzeigt und/oder die Lichtstärke automatisch auf den vorgegebenen Höchstwert zurückgeregelt.

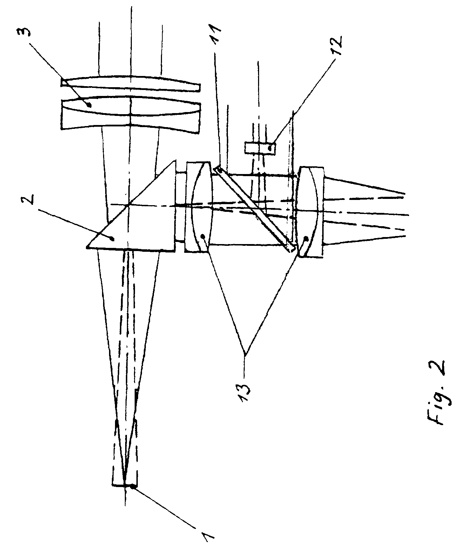

Fig. 2 zeigt eine Anordnung zur Ermittlung und Regelung der Lichtstärke für Spaltlampen oder Spaltleuchtenprojektoren für Geräte mit Doppelachromat (geteilten Objektiven) und dadurch parallelem Strahlengang.
Es sind das Spaltbild 1, das Umlenkprisma 2, das Objektiv Stereomikroskop 3 und die Bestandteile des Doppelachromaten 13 in für Spaltlampen oder Spaltleuchtenmikroskope dieser Bauart bekannter Weise angeordnet. Zwischen den beiden Baugruppen des Doppelachromaten 13 ist in einem Winkel von 45 Grad eine dünne Glasplatte 11 im parallelem Strahlengang angeordnet. Im rechten Winkel zum Strahlengang ist ein Detektor 12 oder eine Detektorbaugruppe wiederum derart angeordnet, daß ein Teil des Strahlenbündels als abgelenktes Strahlenbündel auf den Detektor trifft.

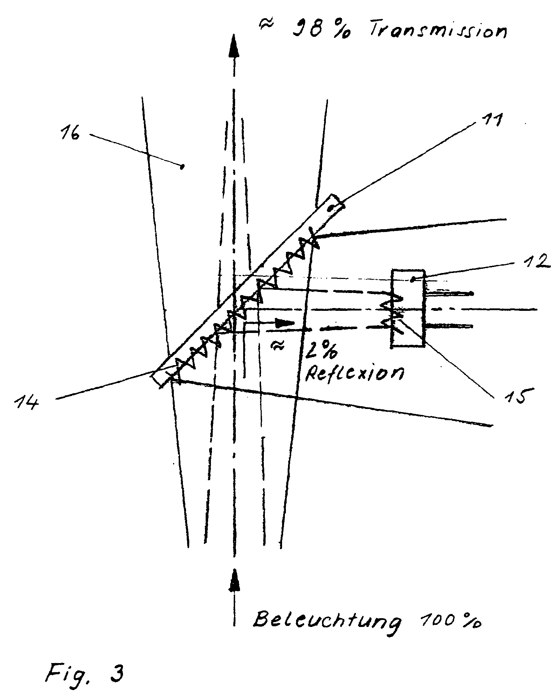

Fig.3 zeigt die Ablenkung eines Teiles der Strahlenbündels durch die dünne Glasplatte 11, die Wendelabbildung 14 auf der dünnen Glasplatte 11, und ein Teil der Wendelabbildung 15 auf dem Detektor 12 sowie die Anordnung der dünnen Glasplatte 11 und des Detektors 12 im Beleuchtungsstrahlengang des Spaltprojektors.

### Aufstellung der verwendeten Bezugszeichen

- 1: Spaltbild
- 2: 90° Umlenkprisma
- 3: Objektiv Stereomikroskop
- 4: Objektiv Spaltprojektor
- 5: Farb-, Neutralglasfilter
- 6: UV-Absorbtionsfilter
- 7: Spaltblende
- 8: Kondensator mit IR-Absorbtion
- 9: Halogenlampe
- 10: Hohlspiegel
- 11: Dünne Glasplatte
- 12: Detektor
- 13: Doppelachromat
- 14: Wendel Halogenlampe
- 15: Teil der Wendelabbildung am Detektor
- 16: Beleuchtungsstrahlengang Spaltprojektor

## Patentansprüche

1. Anordnung zur Ermittlung und Regelung der Lichtstärke für Spaltlampen oder Spaltleuchtenprojektoren mit nichtgeteilten Objektiven der Bauart Stereomikroskop,
**dadurch gekennzeichnet, daß** in einer Spaltlampe oder einem Spaltlampenprojektor eine schrägstehende, dünne Glasplatte (11) mit einer Teilreflexion des Lichtes in einem definierten Winkel zum Strahlengang oberhalb der Filtergruppe, bestehend aus Farbneutralfilter (5) und UV-Absorbtionsfilter (6) zwischen dieser und dem Objektiv Spaltprojektor (4) derart angeordnet ist, daß dadurch ein Teil der auftreffenden Strahlen als abgelenktes Strahlenbündel auf einen seitlich im Gehäuse in einem vom Winkel der dünnen Glasplatte(11) abhängigen Winkel zur Mittelachse des Beleuchtungsstrahlenganges (16) im Gehäuse angeordneten Detektor (12) bzw. eine Detektorbaugruppe trifft, welche die jeweils vorhandene Lichtstärke mißt, die ermittelten Werte zu einer Auswerte- und Steuereinrichtung überträgt, diese die ankommenden Werte mit einem vorgegebenen Höchstwert vergleicht, die Bestrahlungsdosis für das phake oder aphake Auge errechnet und bei Überschreitung des Wertes dieses an einer Anzeige-, Arlarmeinrichtung anzeigt und/oder die Lichtstärke automatisch auf den vorgegebenen Höchstwert zurückregelt.

2. Anordnung zur Ermittlung und Regelung der Lichtstärke für Spaltlampen oder Spaltleuchtenprojektoren für Geräte mit Doppelachromat und dadurch parallelem Strahlengang
**dadurch gekennzeichnet, daß** in einer Spaltlampe oder einem Spaltlampenprojektor eine schrägstehende, dünne Glasplatte (11) mit einer Teilreflexion des Lichtes in einem definierten Winkel zum Strahlengang zwischen den Linsenbaugruppen des Doppelachromaten (13) im Beleuchtungsstrahlengang (16) angeordnet ist, dadurch ein Teil der auftreffenden Strahlen als abgelenktes Strahlenbündel auf eine seitlich in einem vom Winkel der dünnen Glasplatte (11) abhängigen Winkel im Gehäuse angeordneten Detektor (12) bzw. eine Detektorbaugruppe trifft, welche die jeweils vorhandene Lichtstärke mißt, die ermittelten Werte zu einer Auswerte- und Steuereinrichtung überträgt, diese die ankommenden Werte mit einem vorgegebenen Höchstwert vergleicht, die Bestrahlungsdosis für das phakische und aphakische Auge errechnet und bei Überschreitung des Wertes dieses an einer Anzeige-, Arlarmeinrichtung anzeigt und/oder die Lichtstärke automatisch auf den vorgegebenen Höchstwert zurückregelt.

## Claims

1. An arrangement for determining and controlling the luminous intensity of slit lamps or slit lamp projectors with undivided lens systems of the stereomicroscope type,
**characterized by** the fact
that a thin, inclined glass plate (11) which partially reflects the light at a defined angle relative to the beam path is arranged in a slit lamp or a slit lamp projector above the filter group that consists of a neutral color filter (5) and a UV-absorption filter (6), namely between this filter group and the lens system of the slit lamp projector (4), in such a way that part of the incident light beams is incident on a detector (12) or a detector assembly that is arranged laterally in the housing at a relative angle to the central axis of the illumination beam path (16) which depends on the angle of the thin glass plate (11) in the form of a deflected luminous beam, wherein the detector or the detector assembly measures the respective luminous intensity and transmits the determined values to an evaluation and control device, and wherein the evaluation and control device compares the arriving values with a predetermined maximum value, calculates the radiation dose for the phakic or aphakic eye and, if the value is exceeded, displays the exceeding of the value on a display and alarm device and/or automatically controls the luminous intensity such that it is lowered to the predetermined maximum value.

2. The arrangement for determining and controlling the luminous intensity of slit lamps or slit lamp projectors intended for devices with double achromatic lens systems and parallel beam paths,
**characterized by** the fact
that a thin, oblique glass plate (11) which partially reflects the light at a defined angle relative to the beam path is arranged in a slit lamp or a slit lamp projector, namely in the illumination beam path (16) between the lens assemblies of the double achromatic lens systems (13), in such a way that part of the incident beams are incident on a detector (12) or a detector assembly that is arranged laterally in the housing at an angle that depends on the angle of the thin glass plate (11) in the form of a deflected luminous beam, wherein the detector or the detector assembly measures the respective luminous intensity and transmits the determined values to an evaluation and control device, and wherein the evaluation and control device compares the arriving values with a predetermined maximum value, calculates the radiation dose for the phakic or aphakic eye and, if the value is exceeded, displays the exceeding of the value on a display and alarm device and/or automatically controls the luminous intensity such that it is lowered to the predetermined maximum value.

## Revendications

1. Agencement pour la détermination et la régulation de l'intensité lumineuse pour lampe à fente ou projecteur de lampe à fente comprenant des objectifs non divisés du type de ceux d'un stéréomicroscope,
**caractérisé en ce que** dans une lampe à fente ou un projecteur de lampe à fente, une plaque en verre (11) mince, disposée en oblique avec une réflexion partielle de la lumière sous un angle défini par rapport à la marche de rayon en amont du groupe de filtre, qui est constitué de filtre de couleur neutre (5) et de filtre d'absorption UV (6), est agencée entre celui-ci et l'objectif projecteur à fente (4) de telle sorte qu'ainsi une partie des rayons incidents est incident en tant que faisceau de rayon dévié sur un détecteur (12) ou un groupe de construction de détecteur disposé latéralement dans le boîtier sous un angle par rapport à l'axe médian de la marche de rayon d'éclairage (16) dans le boîtier, angle qui dépend de l'angle de la plaque en verre (11) mince, le détecteur mesurant l'intensité de lumière présente à chaque fois, transmettant les valeurs déterminées vers un dispositif d'évaluation et de commande, ce dispositif d'évaluation et de commande comparant les valeurs arrivant à une valeur maximale prédéterminée, calculant la dose d'irradiation pour l'oeil équipé ou non d'une lentille, et lors d'un dépassement de la valeur, indiquant ceci sur un dispositif d'affichage ou d'alerte et/ou réduisant l'intensité lumineuse en la régulant de manière automatique à la valeur maximale déterminée.

2. Agencement pour la détermination et la régulation de l'intensité lumineuse pour des lampes à fente ou des projecteurs de lampe à fente pour des appareils à achromat double et à marche de rayon parallèle à travers celui-ci,
**caractérisé en ce que** dans une lampe à fente ou un projecteur de lampe à fente est disposée une plaque en verre (11) mince, disposée en oblique dans la marche de rayon d'éclairage (16) sous un angle défini par rapport à la marche de rayon entre les groupes de construction de lentille de l'achromat double (13) avec une réflexion partielle de la lumière, de telle sorte qu'une partie des rayons incidents est projeté, en tant que faisceau de rayon dévié, sur un détecteur (12) ou un groupe de construction de détecteur disposé latéralement dans le boîtier sous un angle qui dépend de l'angle de la plaque en verre (11) mince, le détecteur ou le groupe de construction de détecteur mesurant l'intensité de lumière qui est présente à chaque fois, transmettant les valeurs déterminées vers un dispositif d'évaluation et de commande, qui compare les valeurs arrivant à une valeur maximale prédéterminée, qui calcule la dose d'irradiation pour l'oeil équipé ou non d'une lentille et qui, lors d'un dépassement de la valeur, affiche ceci sur un dispositif d'affichage ou d'alerte et/ou réduit l'intensité lumineuse de manière automatique en la régulant à la valeur maximale prédéterminée.
